# EUROPEAN PATENT SPECIFICATION

(11) **EP 1 464 307 B1**
(45) Date of publication and mention of the grant of the patent: **22.08.2007**
(21) Application number: 04251928.0
(22) Date of filing: 31.03.2004
(51) Int. Cl.: A61F 2/44, A61L 27/38, A61L 27/36

(54) **Intervertebral fusion implant**
Zwischenwirbelfusionsimplantat
Implant de fusion intervertébrale

(30) Priority: 31.03.2003 US 405062
(43) Date of publication of application: 06.10.2004
(73) Proprietor: DePuy Spine, Inc., Raynham, MA 02767-0350 (US)
(72) Inventor: Dimauro, Thomas M., Southboro Massachusetts 01772 (US); Serhan, Hassan, South Easton Massachusetts 02375 (US)
(74) Representative: Kirkham, Nicholas Andrew

(56) References cited:
- WO-A-01/70139
- DE-A- 4 409 836
- US-A- 5 197 985
- US-A- 6 037 519
- US-A1- 2003 031 695

## Description

Implantable intervertebral fusion devices are routinely used by surgeons to treat degenerative disc disease, discongenic lower back pain, spondylolisthesis, ruptured discs due to injury and other spinal conditions. Fusion devices are used to keep adjacent vertebrae in the correct spaced apart position while bone growth takes place to complete the fusion of the adjacent vertebrae. Typically, intervertebral fusion devices are hollow cages with side walls made from stainless steel, cobalt or titanium alloy which provide strength to support intervertebral forces. The hollow space between the side walls is usually filled with bone graft material that is either provided by the patient (autogenous) or provided by a third party donor (allogenous).

Unfortunately, the metallic supporting frame of the prior art fusion cages is not osteoconductive and therefore does not form a strong mechanical attachment to a patient's bone tissue. This can lead to graft necrosis, poor fusion and poor stability. In addition, the prior art fusion cages must be filled with autologous bone graft material or allograft bone material. However, the supply of autologous bone material is limited and significant complications can occur from the bone harvesting procedure. Moreover, the patient must undergo two separate incisions which may increase the pain and recuperation time of the patient. Allograft bone material is also in limited supply and carries a risk of disease transmission.

In view of the problems discussed above, a significant need exist for further improvement in the design of spinal fusion devices.

WO 01/70139 A discloses skeletal reconstruction cages, as defined in the preamble of claim 1.

DE 4409836 discloses a device for the mechanical protection of an implant or transplant when being introduced into and/or residing in a living body.

US 2003/0031695 discloses regeneration and augmentation of bone using mesenchymal stem cells.

US 6,037,519 discloses ceramic fusion implants and compositions.

The instant invention relates to an intervertebral fusion device and a method of making the intervertebral fusion device. The intervertebral fusion device can be used to promote fusion between two consecutive vertebrae in a patient. The intervertebral fusion device has an intervertebral fusion cage that has a load bearing wall, and a porous matrix adjacent to the load bearing wall. In a preferred embodiment, the porous matrix is integrally bound to the load bearing wall. The load bearing wall of the fusion cage has a higher compression strength than the internal porous matrix. The open pores of the porous matrix define an inner surface to which one or more agents that promote bone growth arc attached. The agent that promotes bone growth is progenitor cells and is present in a concentration of about 5 times to about 30 times greater than the concentration found in the patient's bone marrow aspirate or platelet rich plasma.

The intervertebral fusion devices are fabricated by passing a solution comprising one or more agents including that promote bone growth through the porous matrix of the fusion cage, thereby attaching the agent to the inner surface.

The intervertebral fusion device can be used to promote fusion of two adjacent vertebrae in a mammal, preferably a human patient. In this method, a solution that has one or more bone growth promoting agents is passed through the intervertebral fusion cage, thereby attaching the agent to the inner surface, then the fusion cage is inserted into the intervertebral space between the two vertebrae of the mammal. In a preferred embodiment, the patient's own bone marrow aspirate or platelet rich plasma is passed through the porous matrix during the surgical procedure to insert the fusion cage. The fusion cage may he inserted into the intervertebral space of a mammal from cither the anterior or posterior side of the mammal using surgical techniques known to those skilled in the art.

One advantage of the intervertebral fusion device of the invention is that the device supplies a concentrated amount of mesenchymal stem cells (MSC's) from the bone marrow aspirate, and so docs not require autologous bone taken from the iliae crest (which requires a second operation) or allograft bone material (which may be in limited supply). In addition, there is no risk disease transmission, as when allograft bone material is used.

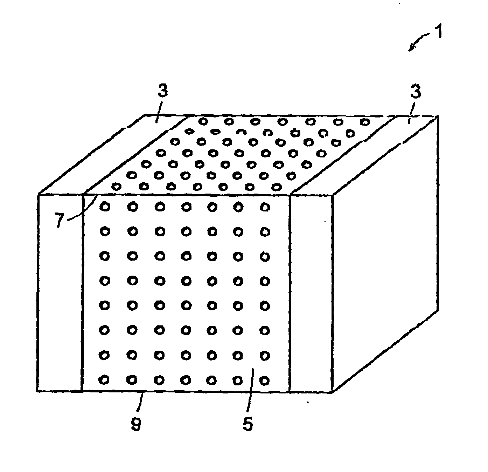

The Figure is a schematic representation of a preferred embodiment of an intervertebral fusion device of the invention.

The present invention relates to a new and improved intervertebral fusion device that has a load bearing wall that can support intervertebral forces and a porous matrix having agents that promote bone growth attached the inner surface of the pores. Typically, the load bearing wall is made of a material that is denser than the porous matrix and can support a load in the range of at least about 8.2 kN (kiloncwlons). This typically requires that the load bearing wall to be made of a material having a compression strength of between about 1000 MPa and 1500 MPa. In a preferred embodiment, the porous matrix is integrally bound to the load bearing wall. The fusion cage may have more than one load bearing wall. In a preferred embodiment, the fusion cage has at least two load bearing walls.

A preferred embodiment of an intervertebral fusion device (1) of the invention is seen in the Figure. The device has two load bearing walls (3) which are adjacent to a porous matrix (5) and an upper surface (7) and a lower surface (9) that will contact the vertebrae to be fused when the device is inserted into the intervertebral space. The device is configured so that the upper and lower surfaces are sufficiently loaded to produce bone growth.

The fusion cage can be made of any biocompatible material and has a suitable size and shape for sealed implantation into the intervertebral space. In some embodiments, the load bearing wall of the fusion cage is approximately parallel to the spinal column when the device is inserted into the intervertebral space. In others, the load bearing wall is angled to produce lordosis. In one embodiment, the fusion cage has a surface at each end of the load bearing wall which will contact the two vertebrae to be fused when the cage is inserted and are approximately perpendicular to the load bearing wall. In another embodiment, the two surfaces that contact the vertebrae to be fused are not perpendicular to the load bearing wall, but instead are tapered in the anterior to posterior direction to achieve lordosis or in the posterior to anterior direction to achieve kyphosis. In a preferred embodiment, the surfaces of the fusion cage that contact the vertebrae to be fused have ridges or teeth to prevent increase the stability of the fusion device once it is inserted into the patient. Optionally, the fusion cage can be a series of cages stacked on top of each other, such as described in U.S. Patent Nos. 6,195,211 and 5,192,327.

Mechanical attachment of the porous matrix to the load bearing will may be achieved by, for example, press fitting a porous matrix cylinder into a hollow sleeve, or by sintering to achieve an integral attachment. Preferably, the load bearing wall and the porous matrix of the fusion cage arc made of the same material. However, the load hearing wall generally will have a density that is greater than the porous matrix. Typically, the load bearing wall will have a porosity in the range of between 0 and about 5 vol%, whereas the porous matrix will typically have a porosity in the range of between about 40 vol% and about 80 vol%. In a preferred embodiment, the pores have an average diameter in the range of between about 25 µm and about 1000 µm. More preferably, the average diameter of the porus is between about 100 µm and about 500 µm.

In one embodiment, the fusion cage of the invention may be made of a synthetic materical, preferably one that is stronger than bone.

Suitable materials for the fusion cage include biopolymers such as, for example, polylactic acid, polyglycolic acid, a copolymer of polylactic acid and polyglycolic acid, a polyarylethyl ketone, polygalactic acid, polycaprolactone, polyethylene oxide, polypropylene oxide, polysulfone, polyethylene, polypropylene, a polyaryletherketone, and combinations thereof. In one embodiment, the fusion cage comprises a polyaryletherketone. In a preferred embodiment, the percentage of the fusion cage that is a polyaryletherketone is in the range of between about 40 vol% and about 90 vol%. In another embodiment, the polyaryletherketone is mixed with carbon fibers which are typically chopped. In a preferred embodiment, the percentage of the fusion cage that is carbon fiber is in the range of between about 1 vol% and about 60 vol%. Examples of polyaryletherketones include polyetheretherketone, poly(arylether ketone ketone), and polyetherketone.

An agent that promotes bone growth is attached to the inner surface of the porous matrix. Agents that promote bone growth include connective tissue progenitor cells (referred to as "progenitor cells" herein) and growth factors. The agent that promotes bone growth is attached to the inner surface, and thereby concentrated, by passing a solution containing the agent through the porous matrix one or more times. Typically, the concentration of the agent that promotes bone growth is increased in the range of between about 2 fold and about 30 fold by passing the solution through the porous matrix of the fusion cage. More preferably, the agent is increased in the range of between about 5 fold to about 20 fold, and more preferably between about 5 fold and 10 fold.

The term "progenitor cells," as used herein, arc cells that are capable of differentiating into cartilage or bone. Examples of progenitor cells include mesenchymal stem cells, hematopoiete cells, and embryonic stem cells. In one embodiment, progenitor cells are attached to the inner surface of the porous matrix by passing bone marrow aspirate suspension through the fusion cage.

As used herein, the term "growth factors" encompasses any cellular product that modulates the growth or differentiation of other cells, particularly connective tissue progenitor cells. Growth factors include, but are not limited to, isoforms of platelet derived growth factors (PDGF), fibroblast growth factors, epithelial growth factors, isoforms of transforming growth factor Beta, insulin-like growth factors, bone morphogenic proteins and precursors thereof. In a preferred embodiment, the growth factor is a bone morphogenic protein or a precursor thereof. In one embodiment, growth factors are attached to the inner surface of the porous matrix by passing platelet rich plasma or bone marrow aspirate suspension through the fusion cage. In another embodiment, growth factors are attached to the inner surface of the porous matrix by passing a solution of recombinant growth factors through the fusion cage.

Bone marrow aspirate contains plasma, nucleated connective tissue progenitor cells, nucleated hematopoietic cells, endothelial cells, and cells derived from contaminating peripheral blood, including red cells and platelets. Since bone marrow aspirate also contains peripheral blood, it is preferred that the bone marrow be collected in a syringe containing an anti-coagulant. Suitable anti-coagulants include, for example, heparin, sodium citrate, and EDTA. Preferably, the bone marrow aspirate is mixed with a sterile isotonic solution to provide a concentration in the range of from about 10 million to about 300 million nucleated cells/ml, preferably from about 20 million to about 250 million nucleated cells/ml, more preferably from about 50 million to about 200 million nucleated cells/ml. Suitable isotonic solutions include, for example, isotonic buffered salt solutions, such as Hank's Balanced Salt Solution and phosphate buffered saline, and tissue culture medium such as minimal essential medium. As used herein, the term "bone marrow aspirate suspension" refers to a bone marrow aspirate that has not been mixed with an isotonic solution and to a bone marrow aspirate that has beco mixed with an isotonic solution.

Platelet rich plasma typically is produced from centrifuging blood and isolating the buffy coat produced therefrom, and may be produced from the blood of the patient receiving the intervertebral fusion device, by methods known to those skilled in the art. Platelet rich plasma contains densely concentrated platelets (which, when activated by thrombin, release growth factors).

In some embodiments, a concentrated fraction of either the BMA or PRP is passed through the porous matrix. Preferably, this concentrated fraction is the buffy coat.

To allow for implantation of the graft into a mammal, it is preferred that the fusion cage be sterile. Preferably, the bone marrow aspirate suspension is permitted to flow through the sterile fusion cage under hydrostatic pressure which may be generated by external forces or by the force of gravity. Preferably, the linear elution rate of the suspension through the fusion cage is between 2 and 500 ml/minute, more preferably between 5 and 200 ml/minute, most preferably between 10 and 100 ml/minute.

Optionally, the effluent is collected sterilely in an effluent collector and recycled through the fusion cage one or more times to increase the number of connective tissue progenitor cells attached to the inner surface of the porous matrix of the fusion cage.

In some embodiments, the device further comprises cell adhesion molecules attached to the inner surface of the porous matrix. These molecules help the device retain the agents passing there through.

Optionally, a wash solution is passed through the fusion cage after the original bone marrow aspirate suspension and any effluents have been passed through the fusion cage. Preferably, the wash solution comprises a sterile, isotonic, buffered solution having a pH range of 7.3 to 7.5. Suitable wash solutions include, for example, phosphate-buffered saline, Hank's balanced salt solution, and minimal essential medium.

Optionally, growth factors or additional cells which secrete or present (i.e. express on their surface) growth factors are attached to the inner surface of the porous matrix prior to use, i.e. before, during or after the time the bone marrow aspirate suspension is passed through the fusion cage. Growth factors which may be added include, for example, isoforms of platelet derived growth factors, fibroblast growth factors, epithelial growth factors, transforming growth factor β, insulin-like growth factor(s), parathyroid hormone (PTH) or PTH related peptide and bone morphogenic proteins and precursors thereof. Preferably, growth factors are added by passing a solution containing the growth factors through fusion cage after all previous suspensions and solutions have been passed through the substrate. Alternatively, grow factors are added by incorporation into the wash solution. Platelets, which are know to secrete growth factors and to adhere to negatively charged surfaces, are added to the graft by passing a suspension of platelets, such as blood or platelet concentrate which contains an anti-coagulant, through the fusion cage.

In devices designed for posterior lumbar interbody fusion (PLIF), the present invention is advantageous over conventional autograft-containing cages in that there is less of a chance that the graft will fall out of the cage during the high-impact insertion of the cage.

### EQUIVALENTS

While this invention has been particularly shown and described with references to preferred embodiments thereof, it will be understood by those skilled in the art that various changes in form and details may be made therein without departing from the scope of the invention encompassed by the appended claims.

## Claims

1. An intervertebral fusion device (1), comprising:
a) an intervertebral fusion cage, comprising
i) a load bearing wall (3); and
ii) a porous matrix (5) mechanically attached to the load bearing wall, wherein the open pores of the porous matrix (5) define an inner surface; and
b) one or more agents that promote bone growth attached to the inner surface **characterised in that** the at least one agent is progenitor cells, wherein the fusion device contains nucleated cells in a range of 400 million cells per millilitre and 6 billion cells per millilitre which comprises the progenitor cells, and further **characterised in that** the fusion cage comprises: a biopolymer or a polyaryletherketone selected from the group consisting of polyetheretherketone, poly(arylether ketone ketone), polyetherketone, and combinations thereof;

2. The device of claim 1, wherein the progenitor cells are selected from the group consisting of mesenchymal stem cells, hematopoietic cells, embryonic stem cells and combinations thereof.

3. The device of claim 1 or claim 2, further comprising a growth factor.

4. The device of claim 3, wherein the growth factor is selected from the group consisting of bone morphogenic protein or a precursor thereof, isoforms of platelet derived growth factors, fibroblast growth factors, epithelial growth factors, isoforms of transforming growth factor Beta, insulin-like growth factors, and combinations thereof, preferably wherein the growth factor is bone morphogenic protein or a precursor thereof.

5. The device of any one of claims 1 to 4, wherein the fusion cage comprises a ceramic.

6. The device of claim 5, wherein the ceramic is an oxide of alumina, zirconia or a combination thereof.

7. The device of claim 6, wherein the ceramic comprises hydroxyapatite, tricalcium phosphate, or a combination thereof.

8. The device of any one of claims 1 to 7, wherein the biopolymer is selected from the group consisting of a polylactic acid, polyglycolic acid, a copolymer of polylactic acid and polyglycolic acid, a polyarylethyl ketone, polygalactic acid, polycaprolactone, polyethylene oxide, polypropylene oxide, polysulfone, polyethylene, polypropylene, a polyaryletherketone and combinations thereof.

9. The device of any one of claims 1 to 8, wherein the fusion cage further comprises carbon fibres.

10. The device of claim 9, wherein the composition of the fusion cage is between about 40% and about 60% polyaryletherketone; and/or the composition of the fusion cage is between about 1 % and about 60% carbon fibre.

11. The device according to any one of the preceding claims, wherein the porous matrix (5) is integrally bound to the load bearing wall (3).

12. The device according to any one of the preceding claims, wherein the porous matrix has a porous volume of between about 40% and about 80%.

13. The device according to any one of the preceding claims, wherein the porous matrix has an average pore diameter of between about 25µm and about 1000µm.

14. The device according to any one of the preceding claims, wherein the load bearing (3) wall has an upper and lower bearing surfaces (7, 9) that have teeth.

15. The device according to any one of the preceding claims, wherein the fusion cage is tapered in the anterior to posterior direction to achieve lordosis.

16. The device according to any one of the preceding claims, wherein the fusion cage is tapered in the posterior to anterior direction to achieve kyphosis.

17. The device according to any one of the preceding claims, wherein the device comprises more than one fusion cage stacked on top of each other.

18. A method of fabricating an intervertebral fusion device (1) according to claim 1, comprising the steps of:
a) providing an intervertebral fusion cage, comprising:
i) a load bearing wall (3); and
ii) a porous matrix (5) mechanically attached to the load bearing wall (3), wherein the open pores of the porous matrix define an inner surface; and **characterised by**
b) passing a solution comprising one or more agents that promote bone growth through the porous matrix, thereby attaching the agent to the inner surface and forming an effluent of the solution, wherein the agent is progenitor cells.

19. The method of claim 18, wherein the solution comprises progenitor cells.

20. The method of claim 18, wherein the progenitor cells are selected from the group consisting of mesenchymal stem cells, hematopoiete cells, embryonic stem cells, and combinations thereof; and/or the solution is platelet rich plasma or bone marrow aspirate suspension.

21. The method of claim 18 or claim 19, wherein the solution comprises a growth factor.

22. The method of claim 21, wherein:
a) the growth factor is selected from the group consisting of bone morphogenic protein or a precursor thereof, isoforms of platelet derived growth factors, fibroblast growth factors, epithelial growth factors, isoforms of transforming growth factor Beta, insulin-like growth factors, and combinations thereof; and/or
b) the growth factor is bone morphogenic protein or a precursor thereof; and/or
c) the solution comprises a recombinant growth factor; and/or
d) the solution is platelet rich plasma or bone marrow aspirate suspension.

## Patentansprüche

1. Eine Bandscheibenverbindungsvorrichtung (1), aufweisend:
(a) einen Bandscheibenverbindungskäfig, aufweisend
i) eine Last aufnehmende Wand (3) und
ii) eine poröse Matrix (5), die mechanisch an der Last aufnehmenden Wand befestigt ist, wobei die offenen Poren der porösen Matrix (5) eine innere Oberfläche definieren und
(b) ein oder mehrere Mittel, die Knochenwachstum vorantreiben, befestigt an der inneren Oberfläche, **dadurch gekennzeichnet, dass** das zumindest eine Mittel Vorläuferzellen sind, wobei die Verbindungsvorrichtung nukleierte Zellen in einem Bereich von 400 Millionen Zellen pro Milliliter bis 6 Milliarden Zellen pro Milliliter enthält, welche die Vorläuferzellen aufweisen, und weiterhin **dadurch gekennzeichnet, dass** der Verbindungskäfig aufweist: ein Biopolymer oder ein Polyaryletherketon, ausgewählt aus der Gruppe bestehend aus Polyetheretherketon, Polyaryletherketonketon, Polyetherketon und Kombinationen daraus.

2. Die Vorrichtung aus Anspruch 1, wobei die Vorläuferzellen ausgewählt werden aus der Gruppe bestehend aus Mesenchymstammzellen, blutbildenden Zellen, Embryostammzellen und Kombinationen daraus.

3. Die Vorrichtung aus Anspruch 1 oder Anspruch 2, weiterhin aufweisend einen Wachstumsfaktor.

4. Die Vorrichtung nach Anspruch 3, wobei der Wachstumsfaktor ausgewählt wird aus der Gruppe bestehend aus knochenmorphogenem Protein oder einem Precursor davon, Isoformen von von Plättchen abgeleiteten Wachstumsfaktoren, Fibroplast-Wachstumsfaktoren, Epithel-Wachstumsfaktoren, Isoformen des transformierenden Wachstumsfaktors Beta, insulinähnlichen Wachstumsfaktoren, sowie Kombinationen davon, wobei der Wachstumsfaktor vorzugsweise ein knochenmorphogenes Protein oder ein Precursor davon ist.

5. Die Vorrichtung aus einem der Ansprüche 1 bis 4, wobei der Verbindungskäfig eine Keramik aufweist.

6. Die Vorrichtung aus Anspruch 5, wobei die Keramik ein Oxid aus Aluminiumoxid, Zirkonoxid oder eine Kombination davon ist.

7. Die Vorrichtung aus Anspruch 6, wobei die Keramik Hydroxyapatit, Tricalciumphosphat oder eine Kombination davon aufweist.

8. Die Vorrichtung aus einem der Ansprüche 1 bis 7, wobei das Biopolymer ausgewählt wird aus der Gruppe bestehend aus einer Polymilchsäure, Polyglykolsäure, einem Copolymer aus Polymilchsäure und Polyglykolsäure, einem Polyarylethylketon, Polygalaktosäure, Polycaprolakton, Polyethylenoxid, Polypropylenoxid, Polysulfon, Polyethylen, Polypropylen, einem Polyaryletherketon, sowie Kombinationen davon.

9. Die Vorrichtung aus einem der Ansprüche 1 bis 8, wobei der Verbindungskäfig weiterhin Kohlenstofffasern aufweist.

10. Die Vorrichtung aus Anspruch 9, wobei die Zusammensetzung des Verbindungskäfigs zwischen etwa 40% und etwa 60% Polyaryletherketon ist und/oder die Zusammensetzung des Verbindungskäfigs zwischen etwa 1% und etwa 60% Kohlenstofffaser ist.

11. Die Vorrichtung gemäß einem der vorhergehenden Ansprüche, wobei die poröse Matrix (5) integral an die Last aufnehmende Wand (3) gebunden ist.

12. Die Vorrichtung gemäß einem der vorhergehenden Ansprüche, wobei die poröse Matrix ein Porenvolumen von zwischen etwa 40% und etwa 80% hat.

13. Die Vorrichtung gemäß einem der vorhergehenden Ansprüche, wobei die poröse Matrix einen mittleren Porendurchmesser von zwischen etwa 25 µm und etwa 1000 µm hat.

14. Die Vorrichtung gemäß einem der vorhergehenden Ansprüche, wobei die Last aufnehmende Wand (3) eine obere und eine untere aufnehmende Oberfläche (7, 9) hat, die Zähne hat.

15. Die Vorrichtung gemäß einem der vorhergehenden Ansprüche, wobei der Verbindungskäfig in der Richtung von vorne nach hinten spitz zulaufend ist, um Lordose zu erreichen.

16. Die Vorrichtung gemäß irgendeinem der vorhergehenden Ansprüche, wobei der Verbindungskäfig in der Richtung von hinten nach vorne spitz zulaufend ist, um Kyphose zu erreichen.

17. Die Vorrichtung gemäß irgendeinem der vorhergehenden Ansprüche, wobei die Vorrichtung mehr als einen Verbindungskäfig aufweist, die aufeinander gestapelt sind.

18. Ein Verfahren zur Herstellung einer Bandscheibenverbindungsvorrichtung (1) gemäß Anspruch 1, aufweisend die Schritte:
a) zur Verfügung stellen eines Bandscheibenverbindungskäfigs, aufweisend:
i) eine Last aufnehmende Wand (3) und
ii) eine poröse Matrix (5), die mechanisch an der Last aufnehmenden Wand (3) befestigt ist, wobei die offenen Poren der porösen Matrix eine innere Oberfläche definieren, charakterisiert durch
b) Hindurchleiten einer Lösung, aufweisend ein oder mehrere Mittel, die Knochenwachstum durch die poröse Matrix vorantreiben, wobei das Mittel an die innere Oberfläche angeheftet wird und einen Ausfluss der Lösung bildet, wobei das Mittel Vorläuferzellen sind.

19. Das Verfahren nach Anspruch 18, wobei die Lösung Vorläuferzellen aufweist.

20. Das Verfahren nach Anspruch 18, wobei die Vorläuferzellen ausgewählt werden aus der Gruppe bestehend aus Mesenchymstammzellen, blutbildenden Zellen, Embryostammzellen, sowie Kombinationen davon, und/oder die Lösung plättchenreiches Plasma oder eine Suspension aus Knochenmarkaspirat ist.

21. Das Verfahren nach Anspruch 18 oder Anspruch 19, wobei die Lösung einen Wachstumsfaktor aufweist.

22. Das Verfahren nach Anspruch 21, wobei:
a) der Wachstumsfaktor ausgewählt wird aus der Gruppe bestehend aus knochenmorphogenem Protein oder einem Presursor davon, Isoformen von von Plättchen abgeleiteten Wachstumsfaktoren, Fibroplast-Wachstumsfaktoren, Epithel-Wachstumsfaktoren, Isoformen von transformiertem Wachstumsfaktor Beta, insulinähnlichen Wachstumsfaktoren und Kombinationen davon und/oder
b) der Wachstumsfaktor knochenmorphogenes Protein oder ein Precursor davon ist und/oder
c) die Lösung einen rekombinanten Wachstumsfaktor aufweist und/oder
d) die Lösung Plättchen angereichertes Plasma oder eine Suspension aus Knochenmarkaspirat ist.

## Revendications

1. Dispositif de fusion intervertébrale (1), comprenant :
a) une cage de fusion intervertébrale, comprenant
i) une paroi de support de charge (3) ; et
ii) une matrice poreuse (5) fixée mécaniquement à la paroi de support de charge, dans laquelle les pores ouverts de la matrice poreuse (5) définissent une surface interne ; et
b) un ou plusieurs agents qui favorisent la croissance osseuse fixés à la surface interne, **caractérisé en ce que** l'au moins un agent est constitué de cellules progénitrices, dans lequel le dispositif de fusion contient des cellules nucléées, dans une plage de 400 millions de cellules par millilitre et 6 milliards de cellules par millilitre, qui comprennent les cellules progénitrices, et **caractérisés en outre en ce que** la cage de fusion comprend : un biopolymère ou un polyaryléthercétone choisi dans le groupe comprenant le polyétheréthercétone, le poly(aryléther cétone cétone), le polyéthercétone et des combinaisons de ceux-ci.

2. Dispositif selon la revendication 1, dans lequel les cellules progénitrices sont choisies dans le groupe comprenant les cellules souches mésenchymateuses, les cellules hématopoïétiques, les cellules souches embryonnaires et des combinaisons de celles-ci.

3. Dispositif selon la revendication 1 ou la revendication 2, comprenant en outre un facteur de croissance.

4. Dispositif selon la revendication 3, dans lequel le facteur de croissance est choisi parmi le groupe comprenant une protéine morphogénique osseuse ou un précurseur de celle-ci, des isoformes de facteurs de croissance d'origine plaquettaire, des facteurs de croissance des fibroblastes, des facteurs de croissance épithéliale, des isoformes de facteur de croissance transformant Beta, des facteurs de croissance de type insuline et des combinaisons de ceux-ci, de préférence dans lesquels le facteur de croissance est une protéine morphogénique osseuse ou un précurseur de celle-ci.

5. Dispositif selon l'une quelconque des revendications 1 à 4, dans lequel la cage de fusion comprend une céramique.

6. Dispositif selon la revendication 5, dans lequel la céramique est un oxyde d'alumine, une zircone ou une combinaison de ceux-ci.

7. Dispositif selon la revendication 6, dans lequel la céramique comprend une hydroxyapatite, un phosphate tricalcique ou une combinaison de ceux-ci.

8. Dispositif selon l'une quelconque des revendications 1 à 7, dans lequel le biopolymère est choisi dans le groupe comprenant un acide polylactique, un acide polyglycolique, un copolymère d'acide polylactique et d'acide polyglycolique, un cétone de polyaryléthyle, un acide polygalactique, un polycaprolactone, un oxyde de polyéthylène, un oxyde de polypropylène, un polysulfone, un polyéthylène, un polypropylène, un polyaryléthercétone et des combinaisons de ceux-ci.

9. Dispositif selon l'une quelconque des revendications 1 à 8, dans lequel la cage de fusion comprend en outre des fibres de carbone.

10. Dispositif selon la revendication 9, dans lequel la composition de la cage de fusion est comprise entre 40 % et 60 % environ de polyaryléthercétone ; et/ou la composition de la cage de fusion est comprise entre 1 % et 60 % environ de fibre de carbone.

11. Dispositif selon l'une quelconque des revendications précédentes, dans lequel la matrice poreuse (5) est liée intégralement à la paroi de support de charge (3).

12. Dispositif selon l'une quelconque des revendications précédentes, dans lequel la matrice poreuse a un volume poreux compris entre 40 % et 80 % environ.

13. Dispositif selon l'une quelconque des revendications précédentes, dans lequel la matrice poreuse a un diamètre de pore moyen compris entre 25 µm et 1000 µm environ.

14. Dispositif selon l'une quelconque des revendications précédentes, dans lequel la paroi de support de charge (3) a des surfaces de support supérieure et inférieure (7, 9) qui ont des dents.

15. Dispositif selon l'une quelconque des revendications précédentes, dans lequel la cage de fusion est effilée dans la direction antérieure à postérieure pour obtenir une lordose.

16. Dispositif selon l'une quelconque des revendications précédentes, dans lequel la cage de fusion est effilée dans la direction postérieure à antérieure pour obtenir une cyphose.

17. Dispositif selon l'une quelconque des revendications précédentes, dans lequel le dispositif comprend plusieurs cages de fusion empilées les unes au-dessus des autres.

18. Procédé de fabrication d'un dispositif de fusion intervertébral (1) selon la revendication 1, comprenant les étapes de :
a) fourniture d'une cage de fusion intervertébrale, comprenant :
i) une paroi de support de charge (3) ; et
ii) une matrice poreuse (5) fixée mécaniquement à la paroi de support de charge (3), dans laquelle les pores ouverts de la matrice poreuse définissent une surface interne ; et **caractérisée par**
b) le passage d'une solution comprenant un ou plusieurs agents qui favorisent la croissance osseuse à travers la matrice poreuse, fixant ainsi l'agent à la surface interne et formant un effluent de la solution, dans lequel l'agent est constitué de cellules progénitrices.

19. Procédé selon la revendication 18, dans lequel la solution comprend des cellules progénitrices.

20. Procédé selon la revendication 18, dans lequel les cellules progénitrices sont choisies parmi le groupe comprenant les cellules souches mésenchymateuses, les cellules hematopoïétiques, les cellules souches embryonnaires et des combinaisons de celles-ci ; et/ou la solution est un plasma riche en plaquettes ou une suspension de ponction de moelle osseuse.

21. Procédé selon la revendication 18 ou la revendication 19, dans lequel la solution comprend un facteur de croissance.

22. Procédé selon la revendication 21, dans lequel :
a) le facteur de croissance est choisi dans le groupe comprenant une protéine morphogénique osseuse ou un précurseur de celle-ci, des isoformes de facteurs de croissance d'origine plaquettaire, des facteurs de croissance des fibroblastes, des facteurs de croissance épithéliale, des isoformes de facteur de croissance transformant Beta, des facteurs de croissance de type insuline et des combinaisons de ceux-ci ; et/ou
b) le facteur de croissance est une protéine morphogénique osseuse ou un précurseur de celle-ci ; et/ou
c) la solution comprend un facteur de croissance recombinant ; et/ou
d) la solution est un plasma riche en plaquettes ou une suspension de ponction de moelle osseuse.
